# EUROPEAN PATENT SPECIFICATION

(11) **EP 3 547 851 B1**
(45) Date of publication and mention of the grant of the patent: **21.04.2021**
(21) Application number: 17829938.4
(22) Date of filing: 30.11.2017
(51) Int. Cl.: A23L 33/135, A61K 35/747

(54) **L. SALIVARIUS SGL03:PROBIOTIC ACTIVITIES AND PRODUCTION OF ANTIMICROBIAL PROTEINS**
L. SALIVARIUS SGL03: PROBIOTISCHE AKTIVITÄTEN UND PRODUKTION VON ANTIMIKROBIELLEN PROTEINEN
L. SALIVARIUS SGL03

(30) Priority: 30.11.2016 IT 201600121481
(43) Date of publication of application: 09.10.2019
(73) Proprietor: Sintal Dietetics S.r.l., 64020 Castellalto (IT)
(72) Inventor: MARINI, Umberto, 64020 Castellnuovo Vomano (IT)
(74) Representative: Zaccaro, Elisabetta
(86) International application number: PCT/EP2017/080923
(87) International publication number: WO 2018/100035

(56) References cited:
- US-A1- 2005 084 482
- Anonymous: "Lac Vis Nature", Wikifarmaco , 8 January 2016 (2016-01-08), XP002770944, Retrieved from the Internet: URL:http://it.wikifarmaco.org/Lac_Vis_Natu re [retrieved on 2017-06-08]
- Anonymous: "Bio-flora capsules", Biodelta , 11 January 2016 (2016-01-11), XP002770945, Retrieved from the Internet: URL:http://www.biodelta.it/en/bio-flora-ca psules/ [retrieved on 2017-06-08]
- E. A. SVETOCH ET AL: "Isolation of Lactobacillus salivarius 1077 (NRRL B-50053) and Characterization of Its Bacteriocin, Including the Antimicrobial Activity Spectrum", APPLIED AND ENVIRONMENTAL MICROBIOLOGY, vol. 77, no. 8, 15 April 2011 (2011-04-15) , pages 2749-2754, XP055011320, ISSN: 0099-2240, DOI: 10.1128/AEM.02481-10
- O'SHEA EILEEN F ET AL: "Bactofencin A, a New Type of Cationic Bacteriocin with Unusual Immunity", MBIO, vol. 4, no. 6, November 2013 (2013-11), pages 1-9, XP002770946, ISSN: 2150-7511
- DATABASE Protein [Online] 3 August 2016 (2016-08-03), Claesson,M.J. et al.: "50S ribosomal protein L27 [Lactobacillus salivarius UCC118]: Multireplicon genome architecture of Lactobacillus salivarius", XP002770947, retrieved from NCBI Database accession no. YP_535847
- DATABASE Protein [Online] 3 August 2016 (2016-08-03), Claesson,M.J. et al.: "DNA-binding protein HU [Lactobacillus salivarius UCC118]: Multireplicon genome architecture of Lactobacillus salivarius.", XP002770948, retrieved from NCBI Database accession no. YP_536662

### SEQUENCE LISTING

<110> SINTAL DIETETICS S.R.L.
<120> L. salivarius SGL03: probiotic activities and production of
   antimicrobial proteins
<130> 16426PTWO
<150> IT102016000121481
   <151> 2016-11-30
<160> 12
<170> BiSSAP 1.3.6
<210> 1
   <211> 20
   <212> DNA
   <213> Artificial Sequence
<220>
   <223> Primer 16S-27 Forward
<400> 1
   agagtttgat cctggctcag 20
<210> 2
   <211> 19
   <212> DNA
   <213> Artificial Sequence
<220>
   <223> Primer 16-1552
<400> 2
   aaggaggtgw tcarccgca 19
<210> 3
   <211> 20
   <212> DNA
   <213> Artificial Sequence
<220>
   <223> Primer L Sal1
<400> 3
   aatcgctaaa ctcataacct 20
<210> 4
   <211> 20
   <212> DNA
   <213> Artificial Sequence
<220>
   <223> Primer L Sal2
<400> 4
   cactctcttt ggctaatctt 20
<210> 5
   <211> 37
   <212> DNA
   <213> Artificial Sequence
<220>
   <223> Primer Forward rpmA
<400> 5
   ctcagcacat atgttaatga atttacaatt cttcgct 37
<210> 6
   <211> 39
   <212> DNA
   <213> Artificial Sequence
<220>
   <223> Primer Reverse rpmA
<400> 6
   ccctcgacat atggataaat taaaagttac tttaattcg 39
<210> 7
   <211> 34
   <212> DNA
   <213> Artificial Sequence
<220>
   <223> Primer Forward rpmD
<400> 7
   gctggatcct tattattcag caacagggta aaca 34
<210> 8
   <211> 34
   <212> DNA
   <213> Artificial Sequence
<220>
   <223> Primer Reverse rpmD
<400> 8
   gatggatccc tactatttag ccaattcaac gtcc 34
<210> 9
   <211> 36
   <212> DNA
   <213> Artificial Sequence
<220>
   <223> Primer Forward rpsT
<400> 9
   ccacctcata tgccaattat caaatctgct attaaa 36
<210> 10
   <211> 33
   <212> DNA
   <213> Artificial Sequence
<220>
   <223> Primer Rev rpsT S20
<400> 10
   tctggatcct tattatttag ctaaacgagc agc 33
<210> 11
   <211> 35
   <212> DNA
   <213> Artificial Sequence
<220>
   <223> Primer Forward LSL_0885
<400> 11
   ttggacgcat atggcaaaca aagcagcatt gattg 35
<210> 12
   <211> 36
   <212> DNA
   <213> Artificial Sequence
<220>
   <223> Primer Rev LSL_0885
<400> 12
   ggcggatcct tattatttaa cagcgtcttt caaaga 36

## Description

### FIELD OF THE INVENTION

The present invention relates to the use of a strain of *L. salivarius SGL03* as an antimicrobial agent.

The invention further concerns a composition comprising *L. salivarius SGL03* and one or more antimicrobial proteins, and the use of such a composition for the preparation of a nutraceutical or dermo-cosmetic product.

### STATE OF THE ART

Probiotics are living microorganisms that can bring beneficial properties to the body, if they are included in adequate quantities in the diet. For an organism to be considered "probiotic", there must be scientific studies that ensure on one hand its safe use in humans and, on the other, the presence of properties such as resistance to the aggression of gastric, pancreatic and bile juices, and the ability to adhere firmly to the intestinal mucosa by colonizing it.

Lactic Acid Bacteria (LAB), for the most part represented by lactobacilli and bifidobacteria, are the most common types of probiotic microorganisms. Such microorganisms may carry out their effect at the level of the host organism, directly or indirectly, by modulating the endogenous ecosystem or the immune response. Some probiotics, on the other hand, may act by directly strengthen the intestinal barrier, preventing permeability, and the subsequent loss of macromolecules, which are observed in intestinal infections and food intolerances, or by exerting a trophic action on the colon mucosa, or by protecting the mucus coating the intestinal wall. Further species indirectly intervene on the intestinal barrier, by stimulating the intestinal immune system, or Gut Associated Lymphoid Tissue (GALT), which constitutes an immune defense barrier for the body. Specifically, probiotics are adjuvants of the endogenous bacterial flora, ensuring the development of IgA producing cells and intestinal epithelial lymphocytes, as well as modulating the production of IgE and interleukins. Basic research also showed that probiotics can act by inhibiting the synthesis of some inflammatory mediators.

The strong antagonistic action that some strains play against pathogenic microorganisms, making them potentially effective in preventing and treating certain pathologies, is widely documented.

The antimicrobial effect is mainly linked to the pH lowering due to the production of lactic acid, while other inhibitory mechanisms comprise hydrogen peroxide production, nutrient competition, and the production of inhibitory metabolites, factors which alone or combined result in a block of pathogens growth.

In the last decades, an important object of study has been the antimicrobial action mediated by the production of "bacteriocins", biologically active peptides, synthesized at ribosomal level, capable of developing linkages to specific anchor sites located on the cell membranes.

The bacteriocin family comprises a wide range of proteins that differ in terms of size, chemical structure, target cells, mode of action, and induced immune mechanisms. It is believed that they are produced by 99% of the bacterial species present in nature.

Bacteriocins produced by Gram-negative bacteria are generally high molecular weight proteins that exhibit a characteristic domain, specific for the adhesion, translocation, or killing activity of the bacteriocin. Bacteriocins produced by Gram-positive bacteria are generally small and thermostable cationic peptides, initially synthesized as pre-peptides and which, following splitting phenomena, are transformed into biologically active molecules.

Their inhibitory spectra may be limited to certain strains belonging to the same species of the producing organism (narrow spectrum), or they can have a broad spectrum of action against various bacteria species (broad spectrum) (Gillor et al., 2005). As for the mechanism of action of these proteins, some of them act forming pores in the cytoplasmic membrane, others interfere with protein synthesis, or have nuclease activity. These substances with strong antimicrobial activity have gained a growing interest for their potential use both in the clinical field, as therapeutics, and in technological/food field as natural preservatives in foods.

Bacteriocins are, in fact, resistant to strong thermal stresses and are active in a wide range of pH. An important peculiarity is then represented by bacteria difficulties in developing resistance to them; a phenomenon linked to their rapid mechanism of action. In addition, being generally sensitive to proteases, they are easily degraded; this means they do not last long in the environment, and therefore the development of resistances by bacteria is less favorable. Finally, bacteriocins are highly specific against clinical pathogens, including multidrug-resistant strains (MDRs, Cotter PD et al., 2013).

Bacteriocins may be used as supplements, or added to probiotics for the treatment of dysbiosis and the restoration of homeostasis in various districts of the body. The probiotic effects of a microbial species are species-characteristic, but they may also vary within the same species.

"Lac Vis Nature" (http://it.wikifarmaco.org/Lac_Vis_Nature) discloses a composition comprising *Lactobacillus Acidophilus* SD5212, *Lactococcus Lactis* ATCC 11454, *Lactobacillus Salivarius* SGL 03, *Bacillus coagulans* 5260, *Lactobacillus Casei* SD5213, *Bifidumbacterium Bifidum* 29521, *Lactobacillus Bulgaricus* SD5589 and further components.

"Bio Flora Capsules" (http://www.biodelta.it/en/bio-flora-capsules/) discloses a food supplement comprising vitamins, minerals and tyndallized lactic ferments and FOS. In the ingredients of the supplement, many different Lactobacilli are listed, such as *Lactobacillus plantarum* SGL 07, *Lactobacillus casei* SGL 15, *Lactobacillus rhamnosus* SGL 06, Lactobacillus acidophilus SGL 11, *Lactobacillus lactis* SGLc01, *Lactobacillus salivarius* SGL 03, *Lactobacillus Delbrueckii* spp *bulgaricus* DSM 20081.

US 2005/084482 describes the use of an *L. salivarius* strain for the prophylaxis or treatment of gastrointestinal inflammatory activity. This inflammatory activity can be due to inflammatory bowel disease, irritable bowel syndrome or cancer.

Svetoch E.A. et al. ("Isolation of Lactobacillus salivarius 1077 (NRRL B-50053) and characterization of its Bacteriocin, including the antimicrobial activity spectrum" (Appl Environ Microbiol 2011 Apr;77(8):2749-54) relates to an *in vitro* anti-*Campylobacter* activity of an *L. salivarius* strain isolate and states that the L. *salivarius* isolate was used for bacteriocin production and enrichment. The polypeptide was purified from the *L. salivarius* isolate and characterized as bacteriocin L-1077.

O'Shea EF et al. ("Bactofencin A, a new type of cationic bacteriocin with unusual immunity" mBio 2013 Oct 29;4(6):e00498-13) relates to Bactofencin A, produced by a porcine intestinal isolated *Lactobacillus salivarius* DPC6502, which is active against pathogenic species including *Staphylococcus aureus* and *Listeria monacytogenes.*

Many *in vitro* experiments have shown that closely related strains may have significantly different adhesion, aggregation, competitive exclusion, and inhibitory activity properties against pathogens. Phenotypic and genotypic characterization is therefore a key requirement for obtaining a probiotic-based product that may adequately be used for health purposes.

The object of the present invention is therefore to provide a specific bacterial strain for the production of antimicrobial proteins with inhibitory activity against harmful microorganisms.

### SUMMARY OF THE INVENTION

The aim of the present work is the identification and the characterization of some antimicrobial proteins produced by the *L. salivarius* SGL 03 DSM 25381 strain, and their therapeutic uses.

The invention therefore concerns the use of *L. salivarius SGL03* DSM 25381 strain for the production of antimicrobial proteins.

In a further embodiment, the invention relates to a probiotic composition comprising *L. salivarius SGL03* DSM 25381, having or being supplemented with one or more antimicrobial proteins selected from the group consisting of 50S Ribosomal Protein L27, DNA-Binding Protein HU, 30S Ribosomal Protein S20, and 50S Ribosomal Protein L30, and optionally magnesium peroxide.

In another aspect, the invention relates to the use of the composition comprising L. *salivarius SGL03* DSM 25381 strain and one or more antimicrobial proteins selected from the group consisting of 50S Ribosomal Protein L27, DNA-Binding Protein HU, 30S Ribosomal Protein S20, and 50S Ribosomal Protein L30 for the preparation of a nutraceutical product.

In yet another aspect, the invention relates to the use of the composition comprising *L. salivarius SGL03* DSM 25381 strain and one or more antimicrobial proteins selected from the group consisting of 50S Ribosomal Protein L27, DNA-Binding Protein HU, 30S Ribosomal Protein S20, and 50S Ribosomal Protein L30 for the preparation of a dermo-cosmetic product.

### DESCRIPTION OF THE FIGURES

The invention will now be described in detail, and with reference to the attached Figures wherein:
Figure 1 depicts the photograph of an agarose gel, wherein a species-specific amplification with primer is shown: B1 and B2 (blanks, DNA-free samples to verify the absence of contamination in the PCR mix), C+ and C- (positive control and negative control with respect to amplified DNA), S (amplified sample corresponding to *L. salivarius SGL03* strain (DSM 25381) by appearance of the 411 pb band as a confirmation of the species), M (molecular weight marker);
Figure 2 depicts the graph of the results of resistance evaluation to high saline concentrations;
Figure 3 depicts the graph of the results of resistance evaluation to acidic environment;
Figure 4 depicts the growth curve of S. *pyogenes* in the presence of various concentrations (1x10⁷CFU/ml, 1x10⁸CFU/ml, 1x10⁹CFU/ml) *of L. salivarius SGL03* strain (DSM 25381), to determine the minimum inhibitory concentration (MIC);
Figure 5 depicts the growth curve of S. *uberis* in the presence of *L. salivarius SGL03* strain (DSM 25381) at the concentration of 1x10⁷CFU/ml (MIC);
Figure 6a shows the growth curve of S. *mutans* in the presence of *L. salivarius SGL03* strain (DSM 25381) at the concentration of 1x10⁷CFU/ml (MIC);
Figure 6b shows the growth curve of S. *mutans,* obtained by testing higher concentrations of *L. salivarius SGL03* strain (DSM 25381) in order to evaluate if the growth inhibition was dose-dependent. The tested concentrations were: 2x10⁷CFU/ml, 3x10⁷CFU/ml, 10x10⁷CFU/ml;
Figure 7 depicts the growth curve of *L. salivarius SGL03* strain (DSM 25381) during the fermentation in batch;
Figure 8 depicts the picture of Tricine SDS-PAGE gel of L. *salivarius SGL03* (DSM 25381) secretome;
Figure 9 depicts the picture of Tricine SDS-PAGE gel of rpmA, rpmD, rpsT, and LSL_0885 expressed in E. coli BL21 (DE) and purified;
Figure 10 depicts the picture of a plate showing the inhibition of S. *pyogenes* growth due to the presence of 15 µl of 1 µg/µl rpmA (A), 15 µl of 1 µg/µl rpmD (B), simultaneous presence (synergistic activity) of 15 µl of 1 µg/µl rpmA and rpmD (C);
Figure 11 depicts the inhibitory effects on S. *pyogenes* ATCC 19615 growth, due to the presence of rpmA (11a, 11b) and rpmD (11c,11d), both tested at the concentration of 133 AU/ml. Specifically, the measurements of the optical density at 600 nm, and the viable cell counts (CFU/ml) are shown: control curve (Δ), growth in the presence of rpmA or rpmD (●).

### DETAILED DESCRIPTION OF THE INVENTION

The invention therefore concerns the use of the *L. salivarius SGL03* strain (DSM 25381) strain as an antimicrobial agent.

In the present invention, when using the definition:
- "bacteriocins" is meant to include a wide range of proteins that differ in terms of size and chemical structure. These biologically active peptides are synthesized at the ribosomal level of various microbial species, and have an antimicrobial action. Surprisingly, said bacteriocins have an inhibitory action against Gram-positive and Gram-negative bacteria.

Specifically, it was found that said bacteriocins have an inhibitory action against bacteria of the *Streptococcus pyogenes, Enterococcus faecium,* and *Streptococcus uberis* species.

In a preferred embodiment, said bacteriocins are selected from the group consisting of Peptidoglycan Binding Protein, 50S Ribosomal Protein L1, 30S Ribosomal Protein S5, 30S Ribosomal Protein S8, 50S Ribosomal Protein L11, 50S Ribosomal Protein L14, 50S Ribosomal Protein L27, DNA-Binding Protein HU, 30S Ribosomal Protein S20, and 50S Ribosomal Protein L30.

In an even more preferred embodiment, said bacteriocins are selected from the group consisting of 50S Ribosomal Protein L27, DNA-Binding Protein HU, 30S Ribosomal Protein S20, and 50S Ribosomal Protein L30.

In another aspect, the invention relates to a probiotic composition comprising *L*. *salivarius SGL03* strain (DSM 25381), having or being supplemented with one or more bacteriocins selected from the group consisting of 50S Ribosomal Protein L27, DNA-Binding Protein HU, 30S Ribosomal Protein S20, and 50S Ribosomal Protein L30.

In a preferred embodiment, the probiotic composition comprises:
- *L. salivarius SGL03* strain (DSM 25381), and
- one or more bacteriocins selected from the group consisting of 50S Ribosomal Protein L27, DNA-Binding Protein HU, 30S Ribosomal Protein S20, and 50S Ribosomal Protein L30,
wherein said one or more antimicrobial proteins are in an amount in a range from 10 to 200µg, 50 to 150µg or preferably from 50 to 100µg of purified protein.

In a preferred embodiment, the probiotic composition comprises:
- *L. salivarius SGL03* strain (DSM 25381);
- one or more bacteriocins selected from the group consisting of 50S Ribosomal Protein L27, DNA-Binding Protein HU, 30S Ribosomal Protein S20, and 50S Ribosomal Protein L30; and
- magnesium peroxide.

The invention may be realized in the form of a food product comprising the composition according to the invention, and other ingredients suitable for food use. Such food products may include the probiotic composition and a food base. The composition or probiotic component may be in liquid or solid form, dried or lyophilized, depending on the needs, and time and temperatures of storage, conservation and transport.

Advantageously, the invention concerns a food product in the form of a dietary supplement.

In comparison to other foods, FSMPs have the ability to fully or partially cover for particular nutritional needs imposed by a disease, disorder or pathological condition, as well as consequences of malnutrition.

Dietary or food supplements are defined as specific products aimed at promoting the intake of certain nutritional principles that are not present in the food of an incorrect diet.

Supplements are recommended in cases where the body is lacking certain alimentary principles: they do not have healing properties, but they supplement a normal diet by completing it.

The probiotic effects are closely related to the microbial species, and there may also be differences within the same species.

Many *in vitro* experiments have shown that closely related strains may have significantly different adhesion, aggregation, competitive exclusion, and inhibitory activity properties against pathogens. Phenotypic and molecular characterization is therefore a key requirement for obtaining a probiotic-based product that may adequately be used for health purposes.

Molecular and phenotypic characterization demonstrated that *L. salivarius SGL03* strain (DSM 25381) possesses the essential characteristics and beneficial properties of a proper probiotic strain that promotes its use in therapeutic formulations.

The invention can be prepared in the form of a nutraceutical product comprising the composition according to the invention, and other ingredients suitable for such a use.

When used as a nutraceutical, the probiotic composition or component may be in liquid or solid form, dried or lyophilized.

In another aspect, then, the invention relates to the use of the composition comprising *L. salivarius SGL03* (DSM 25381), and one or more antimicrobial proteins selected from the group consisting of 50S Ribosomal Protein L27, DNA-Binding Protein HU, 30S Ribosomal Protein S20 and 50S Ribosomal Protein L30, for the preparation of a nutraceutical product.

In a further aspect said nutraceutical is in the form of capsules, tablets, sachets, or chewing gums.

In yet another aspect, the invention relates to the use of the composition comprising *L. salivarius SGL03* strain (DSM 25381), and one or more antimicrobial proteins selected from the group consisting of 50S Ribosomal Protein L27, DNA-Binding Protein HU, 30S Ribosomal Protein S20, and 50S Ribosomal Protein L30, for the preparation of a dermo-cosmetic product.

In another preferred aspect, said dermo-cosmetic is in the form of a patch, a plaster, a cream, a powder or talk, oil, or dry oil.

The following are Examples of embodiments of the present invention provided for illustrative purposes.

### EXAMPLES

### EXAMPLE 1: STRAIN ISOLATION

The strain was isolated from healthy infants' feces.

Initially, a suspension of homogenized fresh stool in 50% de Man Rogosa and Sharpe (MRS) broth (Oxoid, Basingstoke, UK) complex medium, added with 0.05% L-cysteine as reducing agent, was prepared. After incubating the samples at 37°C for 30 minutes, the isolation was carried out in LAMVAB agar (LV) selective medium for vancomycin-resistant lactobacilli. To obtain 1 liter of LAMVAB medium, solution A was prepared dissolving the following components in 500 ml of distilled water:

| | |
|---|---|
| MRS Broth | 52.2 g/l |
| L-Cysteine | 0.25 g/l |
| Green Bromocresol (0.25% in ethanol) | 3 ml/l |

Solution A was brought to pH 5 using 4 N HCl, and sterilized in autoclave at 110°C for 30 minutes. Subsequently, solution B was prepared by resuspending 18 g of agar in 500 ml of distilled water, sterilized at 110°C for 30 minutes. After being cooled down to 55°C, solutions A and B were merged. The agarized medium was added with 20 mg/l vancomycin before being poured into a plate.

### RESULTS

The strain was isolated from healthy infants' feces on LAMVAB (LV) in anaerobiosis, at 37°C for 48 hours. *L. salivarius SGL03* (DSM 25381) colonies resulted to be medium in size, and green in color on LV, while they were cream-white in color on MRS. From a morphological point of view, under a microscope such microorganisms appear in the form of medium length, regular rods.

### EXAMPLE 2: MOLECULAR CHARACTERIZATION

The isolated strain was initially identified by growth on LAMVAB selective medium, as described above, and direct observation of the morphology of colonies grown in plate and cell morphology following observation at the optical microscope.

Certain attribution of the genus and species was done by means of 16S ribosomal DNA analysis techniques and biochemical characterization.

### Analysis of 16S Sequence

The genome of the microorganism was extracted using a commercial kit (Macherey Nagel) following the manufacturer's instructions. For 16S gene amplification reaction, the DNA was used in a 50-100 ng concentration. The amplification reactions were carried out using Biometra T-professional thermocycler (Biometra, Gottingen, Germany). The amplification conditions used were those that gave the best amplification product yields. All reactions were carried out using a final volume of 25 µl.

The components used in all amplification reactions are:
- DyNAzyme (Finnzymes, Espoo, Finland) as thermostable DNA polymerase (1 U per reaction) and its reaction buffer 10, Optimized DyNAzyme buffer (100 mM Tris-HCl, 500 mM KCI, 15 mM MgCl₂, 0.1% Triton X-100, pH 8.8 at 25°C);
- Mixture of triphosphate nucleotides (8 mM, Euroclone, Siziano, Italy).
- set of primers (100 µM, Eurofins MWG Operon, Ebersberg, Germany);
- genomic DNA;
- sterile water.

Below are the primers used, the thermocycle and the expected amplicon.
Primer: 16S-27 For 5'-AGAGTTTGATCCTGGCTCAG-3' SEQ ID NO.1 16-1552 Rev 5'-AAGGAGGTGWTCARCCGCA-3' SEQ ID NO.2
Thermocycle: 95°C 5'

| | | |
|---|---|---|
| 95°C 40" | 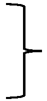 | X40 |
| 55°C 40' | | |
| 72°C 1' | | |
| 72°C 5' | | |
| 15° C ∞ | | |

Amplicon: 1552 bp

The fragment of approximately 1550 bp was sequenced (MWG Biotech, Ebersberg, Germany), and it was aligned against Blast database (NCBI, nucleotide blast) to obtain the correct species identification.

### Species-specific PCR

The same DNA was used as a template for a species-specific primer set for_*L*. *salivarius SGL03* strain (DSM 25381). If the amplified DNA belongs to the species in question, these primers produce a fragment of about 411 bp.

The primers, thermocycle, and amplicon are reported below.
Primer: L Sal1 5'-AAT CGC TAA ACT CAT AAC CT-3' SEQ ID NO.3
L Sal2 5'-CAC TCT CTT TGG CTA ATC TT - 3' SEQ ID NO.4 Thermocycle: 95°C 5'

| | | |
|---|---|---|
| 95°C 1' | 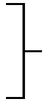 | X35 |
| 55°C 30" | | |
| 72°C 1' | | |
| 72°C 5' | | |
| 15° C ∞ | | |

Amplicon: 411 bp

The visualization of the amplification products was performed by electrophoresis on agarose gel (1-2%). This analysis was carried out by loading on the gel a 10-15 µl volume of the reaction mixture in parallel with the molecular weight marker (MassRuler™ Low Range DNA Ladder, Fermentas).

### Strain Profile in MALDI-TOF MS

To obtain a Maldi-Tof identification profile, the sample was prepared as follows: a bacterial culture of *L. salivarius SGL03* strain (DSM 25381) grown overnight was harvested in order to have a sufficient amount of cells to be detected (5 billions). The cell pellet was washed with physiological solution to remove fermented medium residues, and resuspended in 300 µl of distilled water and 900 µl of pure ethanol. The suspension was well homogenized. The sample was then centrifuged at 13,000 rpm for 2 minutes, and allowed to dry in the air after removing the supernatant. The sample was stored at -20°C while waiting for the analysis.

At the time of the analysis, the sample was resuspended with 50 µl of 70% formic acid. After the resuspension, additional 50 µl of acetonitrile were added and it was all vigorously stirred. The sample was then centrifuged at 13,000 rpm for 2 min. 1 µl of the supernatant was placed in the center of a spot in a disposable MALDI plate, and air-dried. The addition of 1 µl of a matrix made of organic materials (a-cyano-4-hydroxycinnamic acid) determined the start of the Maldi Tof analysis.

Maldi Tof MS was performed with a MicroFlex LT system by using the conditions set out in the user manual. The ions generated by the nitrogen laser at the wavelength of 337 nm were linearly captured in a mass range of 2 to 20 KDa. The instrument was connected to Biotyper 2.0 software (Bruker Daltonics) which is able to capture incoming data, process them into mass spectra, and compare them with reference spectra stored in an internal database (3,740 spectra of 319 genera and 1,946 different species). For each MALDI plate containing 24 samples, a standard test was included to calibrate and validate the analysis.

The degree of correspondence with the typical spectrum of each microorganism in the database determines the attribution of a score value expressing the degree of certainty of the proposed identification for the species under consideration. Scores over 2,000 are considered reliable in species-level identification, values ranging from 1,700 to 1,900 are, instead, attributable to a certain gender-specific identification.

The strain was analyzed in triplicate using 3 different bacterial cultures.

### Gram Staining

Gram staining is a type of differential staining that allows to classify bacteria in Gram-positive and Gram-negative.

This identification test, which was carried out using the Gram Color Kit (Liofilchem), is divided into 4 steps, spaced by washings with distilled water, each of which involves treatment with a particular reagent that is left to act for about 1 minute on the surface of a slide on which a microbial colony from a solid soil has been deposited, diluted with a drop of sterile water. The suspension thus obtained is then heat fixed e with a Bunsen burner.

The reagents used, in order of application, are:
- crystal violet,
- Lugol's solution,
- decolorizing solution (ethyl alcohol),
- safranin.

At the end, Gram-positive bacteria appear purple in color, while Gram-negative bacteria are red or pink.

### Catalase Assay

This assay is used to verify the presence of the catalase enzyme in a bacterial culture able to detoxify hydrogen peroxide (Brock *et al.* 1995).

A loop of cells from a solid culture were dissolved in a drop of 3% hydrogen peroxide. The immediate appearance of effervescence is indicative of the presence of catalase.

Effervescence is due to the oxygen that develops in the reaction:

2 H²O₂ → 2 H²O+O₂

### Oxidase Assay

The oxidase assay is an enzymatic test used to detect the presence of cytochrome oxidase enzyme in bacteria. It is positive for aerobic and facultative anaerobic bacteria. Oxidase Test Discs (Liofilchem), i.e. paper discs of 3 mm diameter impregnated with tetramethyl-p-phenylenediamine hydrochloride (Kovacs reagent), were used. The disc was moistened with 2 drops of distilled water, and rubbed with a loop of a pure colony.

The development of a blue color within 30 seconds demonstrates the oxidation of the substrate contained in the disk. In this case, the microorganism is oxidase-positive, conversely, if no color development occurs, the microorganism will be oxidase-negative.

### Sugar Fermentation Profile (API)

For the biochemical characterization of *L. salivarius SGL03* strain (DSM 25381), the API 50 CH gallery system was used with API 50 CHL (BioMérieux, Milano, Italy) inoculum medium. The gallery consists of 50 microtubes containing dehydrated substrates for the detection of the enzymatic activity and the fermentation of sugars. The bacterial suspension, with a turbidity of 2 McFarland, was distributed in the microtubes, and anaerobiosis was obtained by covering it with sterile paraffin oil. The metabolism of the substrate by the bacterium during the incubation period at 37°C is highlighted by a color change of the medium, following acidification of the medium. Reaction reading was performed on the basis of a reading table where the results were recorded, and the identification was performed using a computer software (www.apiweb.biomerieux.com).

### RESULTS

### Molecular Characterization

Lactobacilli strains selected by isolation with LV medium were taxonomically classified by the study of the entire 16S gene. A 1500 bp long region was amplified using generic primers for eubacteria, sequenced and then aligned using BLAST with public databases. The results enabled a certain classification. The Maldi Tof results shown below provided additional support to the strain identification.

| | | | | | |
|---|---|---|---|---|---|
| **F2(++)(C)** | **11** | **Lactobacillus salivarius** | **2.123** | **Lactobacillus salivarius** | **1.755** |

The *L. salivarius SGL03* strain was deposited on 21/11/2011 at the European International Collection DSMZ, which possess IDA (International Depositary Authority) status, with a "patent deposit" having the following registration number: L. *salivarius SGL03 DSM.25381.*

In addition, the microorganism was amplified with species-specific primers, verifying the positivity by the appearance of the amplicon having the expected 411 pb size.

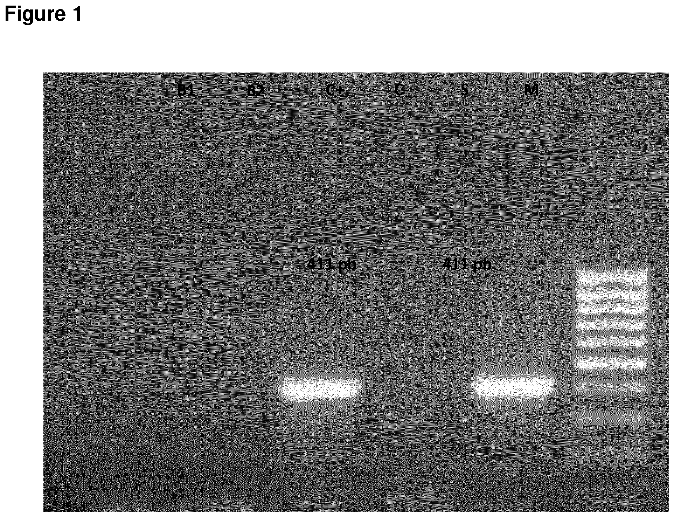

This additional data allows us to assign unambiguously the species, and to have an instrument for controlling and monitoring the culture under examination during the fermentation and lyophilization phases. Figure 1 shows the result of *L. salivarius SGL 03* strain (DSM 25381) amplification using species-specific primers.

### Biochemical Characterization

The *L. salivarius SGL03* strain (DSM 25381) was also analyzed in terms of biochemical profile by Gram staining, catalase test, oxidase test, and API 50CH system.

The results obtained confirmed that the strain is Gram-positive, catalase and oxidase negative, and ferments the sugars as shown in Table 1.

**Table 1: Biochemical characterization of L. salivarius SGL03 by API 50CH system.**

| | |
|---|---|
| **Arbutin** | NEG |
| **Amygdalin** | NEG |
| **N-acetylglucosamine** | POS |
| **Methyl-α-D-glucopyranose** | NEG |
| **Methyl-α-D-mannopyranose** | NEG |
| **D-sorbitol** | POS |
| **D-mannitol** | POS |
| Inositol | NEG |
| **Dulcitol** | NEG |
| L-rhamnose | NEG |
| L-sorbose | NEG |
| D-mannose | POS |
| D-fructose | POS |
| D-glucose | POS |
| D-galactose | POS |
| Methyl-p-D-xylopyranose | NEG |
| Adonitol | NEG |
| L-xylose | NEG |
| D-xylose | NEG |
| D-ribose | NEG |
| L-arabinose | NEG |
| D-arabinose | NEG |
| Erythritol | NEG |
| Glycerol | NEG |
| Control | NEG |
| Potassium 5-ketogluconate | NEG |
| Potassium 2-ketogluconate | NEG |
| Potassium gluconate | NEG |
| L-arabitol | NEG |
| D-arabitol | NEG |
| L-fucose | POS |
| D-fucose | NEG |
| D-tagatose | NEG |
| D-Iyxose | NEG |
| D-turanose | NEG |
| Gentiobiose | NEG |
| Xylitol | NEG |
| Glycogen | NEG |
| Starch | NEG |
| D-raffinose | POS |
| D-melezitose | NEG |
| Inulin | NEG |
| D-trealose | POS |
| D-sucrose | POS |
| D-melibiose | POS |
| D-lactose | POS |
| **D-maltose** | POS |
| D-cellobiose | NEG |
| Salicin | NEG |
| Esculin | NEG |

### EXAMPLE 3: CHARACTERIZATION OF THE PROBIOTIC CHARACTERISTICS OF L. SALIVARIUS SGL03

### Tolerance to the gastro-intestinal environment

In order to evaluate the tolerance to gastro-intestinal juices, vitality over time was determined by plate count of the isolated strain, after it had been contacted with:
- synthetic pepsin;
- synthetic pancreatin;
- various concentrations of bile salts;
- various concentrations of NaCl;
- growth medium acid pH.

### - Pepsin Resistance Test

A pepsin solution consisting of 3 g/l of pepsin, derived from pig gastric mucosa (Sigma-Aldrich, St. Louis, MO), was obtained by solubilizing powdered pepsin in sterile 0.5% NaCl (w/v) saline solution, and the pH was adjusted to 3. Starting from cultures grown overnight in 10 ml of MRS broth medium supplemented with 0.05% of L-cysteine at 37°C for about 18 hours, in the presence of O₂, about 10⁹ cells/ml were harvested by centrifugation at 4,000 rpm for 10 minutes. Subsequently, the pelletized cells were washed with a 0.09% NaCl (w/v) sterile saline solution, and subjected to the same centrifugation cycle. Washing was repeated a second time under the same conditions.

To simulate the passage through the gastric tract, cells were resuspended in 10 ml of pepsin solution at pH 3. The suspension thus prepared was incubated in anaerobiosis, at 37°C, for 2 hours, by measuring the number of CFU/ml at 0, 90 and 120 minutes by plate count.

To monitor growth even in the absence of pepsin, the strain was inoculated into two 0.5% (w/v) sterile saline solutions, at pH 3, one containing pepsin and one without it (control sample). The samples were incubated under the same conditions described above.

The resistance of the treated strain was expressed as a percentage of survival over the control.

### -Pancreatin Resistance Test

A pancreatin solution, consisting of 1 g/l pancreatin from pig pancreas (Sigma-Aldrich), was obtained by solubilizing the powder in 0.5% NaCl (w/v) sterile saline solution, and the pH was adjusted to 8. Starting from cultures grown overnight at 37°C for about 18h, in the presence of O₂, in 10 ml of MRS broth medium supplemented with 0.05% of L-cysteine, about 10⁹ cells/ml were harvested by centrifugation at 4,000 rpm for 10 minutes. Using the same centrifugation cycle, the cells were washed with a 0.09% NaCl (p/v) sterile saline solution.

Subsequently the cells were washed with a sterile neutralization buffer (PBS - 8 g/l NaCl, 0.2 g/l KCI, 1.44 g/l Na₂HPO₄, 0.24 g/l KH₂PO₄) at pH 7 and, to simulate passage through the intestine, they were resuspended in 10 ml of pancreatin solution at pH 8. The suspension was incubated in anaerobiosis, at 37°C for 4 hours, and the number of CFU/ml at 0, 60 and 240 minutes was estimated by plate count. To monitor growth in the absence of pancreatin, the strain under evaluation was inoculated into two 0.5% (w/v) sterile saline solutions, pH 8, one containing pancreatin and the other without it (control sample). The samples were incubated under the same conditions described above.

The resistance of treated strains was expressed as a percentage of survival over control.

### - Resistant Test to Bile Salts, pH and NaCl

The analysis was carried out on bacterial cultures grown overnight. Approximately 10⁹ cells/ml were harvested by centrifugation at 4,000 rpm for 10 minutes. The cells were then washed with 2 ml of sterile physiological solution (NaCl 9 g/l) and, following centrifugation at 4,000 rpm for 10 minutes, they were resuspended in MRS broth containing:
- 0%, 0.5%, 1%, 2%, 3% bile salts (p/v, Sigma-Aldrich);
- 0%, 2%, 6%, 10% NaCl (p/v, Oxoid);
- pH 6.3, 3.5, 2, 1.5, adjusted with 4 N HCl.

The solutions were incubated in anaerobiosis, at 37°C for 24h, for each inoculum the optical density at 600 nm was recorded at 0h, 2h and 24h, while the estimate of the CFU/ml number was performed at 0h and 24h, by plate count.

### Adhesion to Intestinal Cells Monolayer

To assess the adhesion activity of the *L. salivarius SGL03* strain (DSM 25381) strain to the HT29 human enteric line, 1 x 10⁸ bacterial cells were added to an epithelial monolayer of HT29 cells (1.23 x 10⁶ cells) and incubated for 1.5 h. At the end of the incubation, the cell monolayer was washed 4 times with PBS, and the adherent bacterial cells were quantified by Real Time PCR using the genera-specific primer *(*Candela M, Seibold G, Vitali B, Lachenmaier S, Eikmanns BJ, Brigidi P. Real-time PCR quantification of bacterial adhesion to Caco-2 cells: competition between bifidobacteria and enteropathogens. Res. Microbiol. (2005) 156:887-895*).*

The adherent enteropathogens *Salmonella Typhimurium* and *E.coli* ETEC H10407 were used as positive controls, while the non-adherent E. *coli* B44 strain was used as negative control.

### Inhibitory Activity against the Growth of Pathogenic Streptococci Residing in the Oral Cavity

The inhibitory activity of the *L. salivarius SGL03* strain (DSM 25381)_strain against certain streptococci residing in the oral cavity, some associated with pathological process of the oral cavity, was assessed. Specifically, the object of the study was S. *pyogenes,* the most common etiologic agent of pharyngitis and tonsillitis, and S. *mutans,* the primary species responsible for dental caries.

In addition, the antagonistic activity against another streptococcus commensal of the oral mucosa, S. *uberis,* not known as oral pathogen but occasionally associated with genitourinary urinary tract infections, was evaluated.

In order to determine the minimum concentration of *L. salivarius SGL03* strain (DSM 25381) able to inhibit each pathogen growth, a preliminary study on S. *pyogenes* was performed, by testing three different concentrations of *L. salivarius* (1x10⁷ CFU/ml, 1x10⁸ CFU/ml, 1x10⁹ CFU/ml).

For the inhibition test, the starting point was a tube containing 10 ml of M17 medium (Liofilchem), inoculated with S. *pyogenes.* The culture was incubated in aerobiosis at 37°C overnight, under static conditions.

After 24 hours, 4 flasks containing 100 ml of M17 medium were inoculated with 1 ml of S. *pyogenes* culture grown overnight. 1 flask was used as S. *pyogenes* growth control, the other 3 flasks were added with different concentrations of *L. salivarius SGL03* strain (DSM 25381) (1x10⁷CFU/ml, 1x10⁸ CFU/ml, 1x10⁹ CFU/ml). All the flasks were incubated in aerobiosis at 37°C, under static conditions. The microbial growth was assessed at different times by measuring the optical density of the cultures, and calculating the CFU/ml plate count on agarized M17 medium. To differentiate the pathogenic colonies from the *L. salivarius SGL03* strain (DSM 25381) ones, counts were also performed on plates of lactobacilli-specific MRS agar medium. The plates used during the counts were incubated in aerobiosis .at 37°C. for 24-48 hours.

The minimum concentration of *L. salivarius SGL03* strain (DSM 25381) able to inhibit S. *pyogenes* growth was then tested on S. *mutans* and S. *uberis,* in order to verify whether the determined dose was equally effective.

The inhibition test on S. *mutans* and S. *uberis* was performed following the same experimental plot of the preliminary test performed on S. *pyogenes.*

### Antibiotic Resistance Profile

The sensitivity to antimicrobial agents of the *L. salivarius SGL03* strain (DSM 25381) was tested using MIC Test Strip (Liofilchem), a quantitative method for the determination of the minimum inhibitory concentration (MIC) of a single antimicrobial agent against microorganisms. The kit consists of paper strips impregnated with a predetermined gradient of antibiotic concentrations, consisting of 15 dilutions in the range of dilutions used in conventional methods for MIC determination.

The cultures in stationary phase were diluted in MRD buffer (Liofilchem) in order to have a turbidity equal to McFarland 3 standard. 100 µl of this solution were spatulated on the surface of MRS agar medium supplemented with 0.05% of L-cysteine in plate, in order to produce an homogeneous growth. After allowing the agar surface to dry completely, the strip was placed in the center of the plate with the MIC values facing upward. The plates thus prepared were incubated, with the lid facing upward, in aerobiosis, at 37°C for 48 hours.

When the kit strip is applied to the inoculated surface of plated agarized medium, the pre-defined gradient is released by the strip to the medium, therefore, after the incubation, an elliptical, symmetrical, and centered along the strip inhibition area can be observed. The MIC value, expressed in µg/ml, was read at the intersection point between the edge of the inhibition ellipse and the paper strip.

### RESULTS

### Tolerance to the Gastro-intestinal Environment

The microorganism was tested to ensure its gastro-intestinal transit and survival, and ensure it reaches in full vitality the intestinal section where it must exert its physiological activity.

This strain was then tested under the following environmental conditions:
1. pepsin resistance in acidic environment
2. pancreatin resistance in basic environment
3. resistance to high concentrations of bile salts
4. resistance to acidic environment
5. resistance to high saline concentrations

*L. salivarius SGL03* strain (DSM 25381) showed a good tolerance to the gastro-intestinal environment. Specifically, the results showed a good survival rate after 1h, and even after 4h incubation in the presence of pancreatin at pH 8. Similar results were obtained after 1 hour and 30 minutes, and after 2 hours and 30 minutes incubation in the presence of pepsin at pH 3. The results obtained are shown in Table 2 below.

**Table 2: L. salivarius SGL03 resistance to pepsin and pancreatin**

| | **LOG10 CFU/ml** *L. salivarius SGL03* | | |
|---|---|---|---|
| | | t0 | 6.38±0.27 |
| | | t90' | 5.85±0.28 |
| | Test | **t120'** | 6.18±0.25 |
| | | **% Survival 90'** | 91.61±5 |
| **PEPSINA** | | **% Survival 120'** | 96.80±6 |
| **pH 3** | | **t0** | 6.04±0.26 |
| | | **t90'** | 5.30±0.28 |
| | Control | **t120'** | 5.00±0.27 |
| | | **% Survival 90'** | 87.75±7 |
| | | **% Survival 120'** | 82.76±5 |
| | | **t0** | 8.23±0.02 |
| | | **t60'** | 8.07±0.04 |
| | **Test** | **t240'** | 8.03±0.01 |
| | | **% Survival 60'** | 98.13±6 |
| **PANCREATIN** | | **% Survival 240'** | 97.60±5 |
| **pH 8** | | **t0** | 8.33±0.02 |
| | | **t60'** | 8.15±0.01 |
| | **Control** | **t240'** | 7.98±0.03 |
| | | % **Survival 60'** | 97.89±7 |
| | | % **Survival 240'** | 95.78±5 |

The microorganism is slightly affected by the presence of bile salts at concentrations higher than 2% (about 10 times higher than the physiological ones), still showing good survival even in the presence of 3%. Results are shown in Table 3. This makes it suitable for use as a probiotic.

**Table 3: L. salivarius SGL03 resistance to bile salts**

| | **LOG10 (CFU/ML)** | | |
|---|---|---|---|
| **Bile salts** | **t0** | **t 24h** | **Growth** |
| **0%** | 7.62±0.26 | 8.90±0.28 | Normal |
| **0.5%** | 7.77±0.25 | 8.16±0.25 | Normal |
| **1%** | 7.58±0.27 | 8.20±0.28 | Normal |
| **2%** | 7.42±0.28 | 6.82±0.27 | Slight decrease |
| **3%** | 7.51±0.27 | 6.81±0.26 | Slight decrease |

### Resistance in Saline Environment

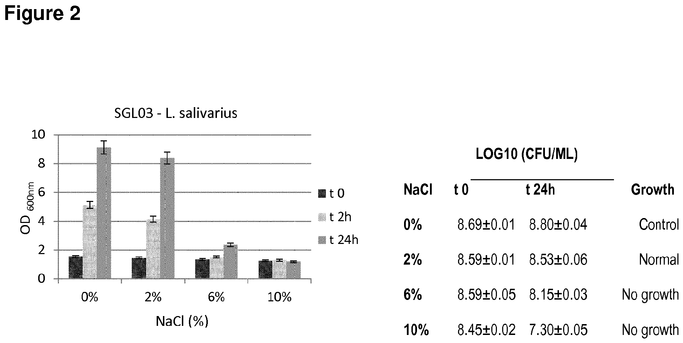

As shown in Figure 2, *L. salivarius SGL03* strain (DSM 25381) is affected by the presence of NaCl in the culture medium. Its survival is ensured up to 2% NaCl; at higher concentrations growth of the microorganism stops.

### Resistance in Acidic Environment

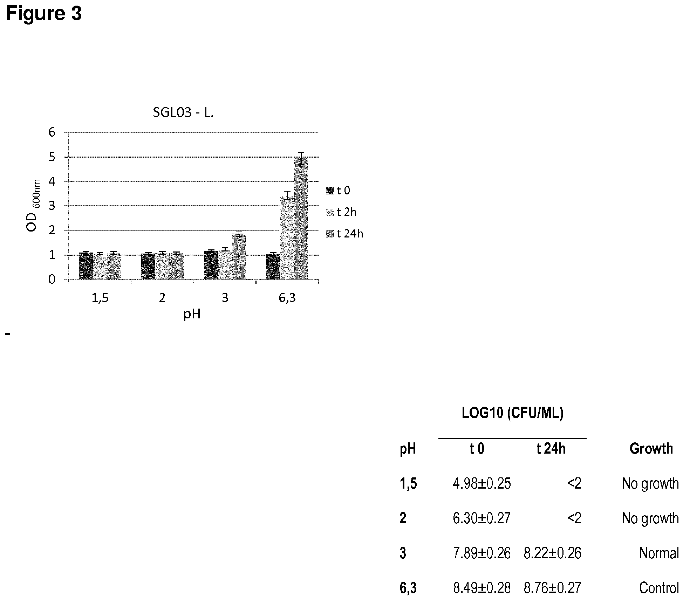

As shown in Figure 3, *L. salivarius* SGL03 strain (DSM 25381) shows a low tolerance to acidic pH; at pH below 3 its survival is compromised.

### Adhesion to the Intestinal Cells Monolayer

The adhesion activity of the *L. salivarius SGL03* strain (DSM 25381) strain to the HT29 human enteric line was evaluated by counting the number of microorganisms adherent to HT29 cells by using Real-Time PCR. The results obtained was of 9.57 bacteria per 100 HT29 cells, therefore *L. salivarius SGL03* strain (DSM 25381) may not be considered an adhesive strain (adhesion reference value, 6.77E+5 Salmonella/100 HT29 cells and 157 E. coli B44/100 HT29).

### Inhibitory Activity on the Growth of Pathogens Residing in the Oral Cavity

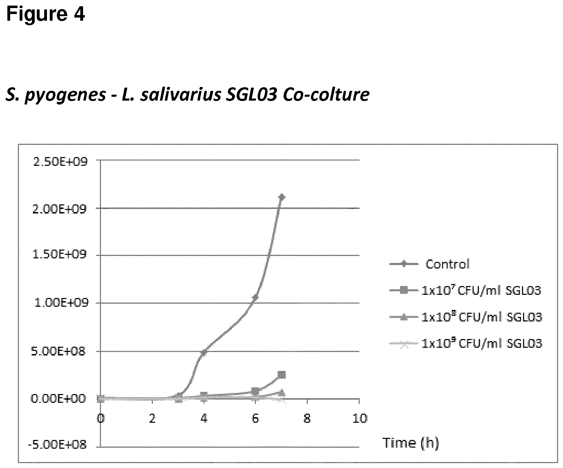

The results of the preliminary study performed on S. *pyogenes* show that *L. salivarius SGL03* strain (DSM 25381) is able to inhibit S. *pyogenes* growth at all concentrations tested (1x10⁷CFU/ml, 1x10⁸ CFU/ml, 1x10⁹ CFU/ml), with a maximum inhibition in the first 6-7 hours of co-culture, after which the microorganism, while being inhibited, begins to slowly grow, as shown in Figure 4. The minimum inhibitory concentration of *L. salivarius SGL03* strain (DSM 25381), determined in the test performed on S. *pyogenes,* is 1x10⁷CFU/ml, and the same concentration was therefore tested on the other oral streptococci under study, S. *uberis* and S. *mutans.* The inhibition test was performed following the same experimental plot used for S. *pyogenes,* and the microbial growth was also evaluated after 24 hours incubation.

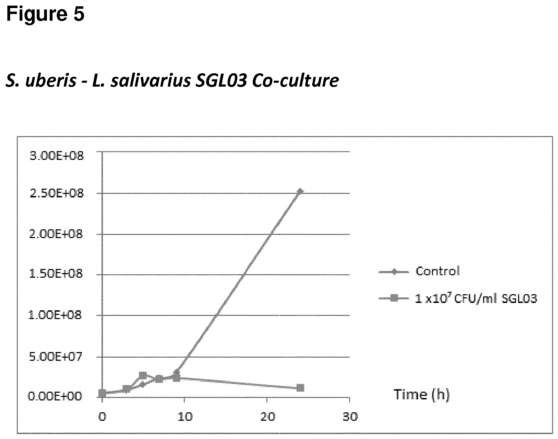

The results showed that, similarly to S. *pyogenes, S. uberis* is inhibited when 1x10⁷CFU/ml of *L. salivarius SGL03* strain (DSM 25381) are present in the growth medium. In addition, the growth inhibition is maintained throughout the 24-hour period, as it is deduced from the graph in Figure 5.

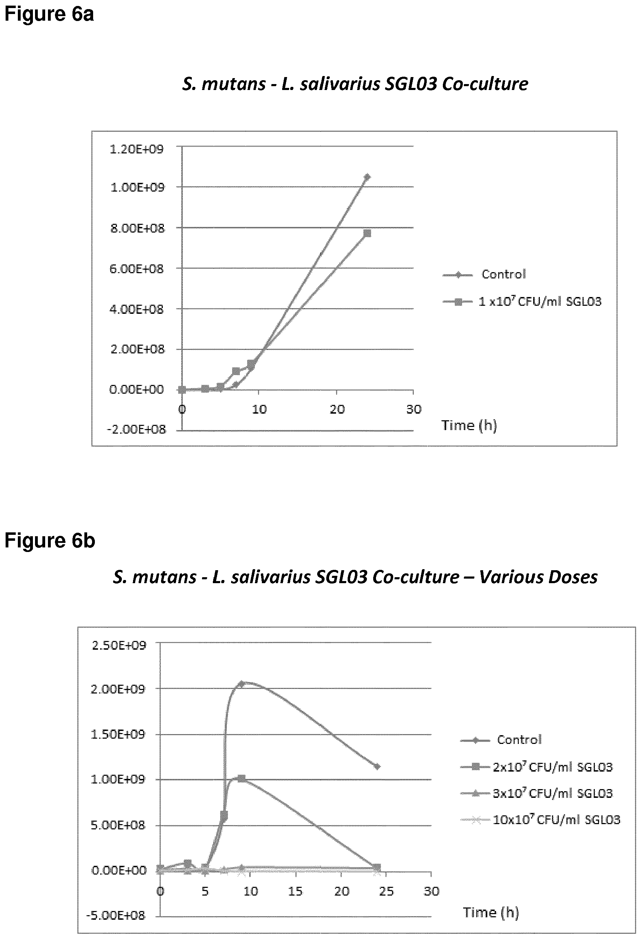

S. *mutans,* on the other hand, is not affected by the presence of *L. salivarius SGL03* strain (DSM 25381) in the culture medium at a concentration of 1x10⁷CFU/ml; in fact, as it can be seen from the graph in Figure 6a, S. *mutans* growth curve shows roughly the same pattern both in the sample and the control. Therefore, a further test at higher concentrations of *L. salivarius SGL03* strain (DSM 25381) (Figure 6b) was performed, in order to evaluate whether the growth inhibition for S. *mutans* was dose-dependent. The tested concentrations were 2x10⁷CFU/ml, 3x10⁷CFU/ml, 10x10⁷CFU/ml.

Th test results are shown after those for the test performed with 1x10⁷CFU/ml SGL03. The minimum concentration of *L. salivarius SGL03* strain (DSM 25381) needed to inhibit the pathogen growth resulted to be 3x10⁷CFU/ml, while at 2x10⁷CFU/ml concentrations the pathogen seems to be little affected by the presence of *L. salivarius SGL03* strain (DSM 25381), especially during the first 8-9 hours of incubation.

### Antibiotic Resistance Profile

Table 4, reported below, shows the resistance profile to antibiotics tested against the *L. salivarius SGL03* strain, by using the MIC Test Strip.

**Table 4: L. salivarius SGL03 antibiotic resistance profile**

| **ANTIBIOTIC** | **FAMILY** | **MECHANISM OF ACTION** | **MIC (µg/ml)** |
|---|---|---|---|
| Amoxicillin | β-lactam | Cell wall synthesis inhibitor | 0.125 |
| Ampicillin | β-lactam | Cell wall synthesis inhibitor | 0.25 |
| Clindamycin | Lincosamide | Protein synthesis inhibitor | 0.094 |
| Chloramphenicol | Macrolide | Protein synthesis inhibitor | 1.5 |
| Ciprofloxacina | Quinolone | DNA synthesis inhibitor | 4 |
| Erythromycin | Macrolide | Protein synthesis inhibitor | 0.19 |
| Gentamycin | Aminoglycoside | Protein synthesis inhibitor | 24 |
| Kanamycin | Aminoglycoside | Protein synthesis inhibitor | >256 |
| Rifampicin | Rifampicin | Protein synthesis inhibitor | 0.064 |
| Streptomycin | Aminoglycoside | Protein synthesis inhibitor | 192 |
| Sulfamethoxazole | Sulfamide | DNA synthesis inhibitor | >1024 |
| Tetracycline | Tetracycline | Protein synthesis inhibitor | 1 |
| Vancomycin | Glycopeptide | Cell wall synthesis inhibitor | >256 |

### EXAMPLE 4: DESCRIPTION OF THE FERMENTATION AND LYOPHILIZATION PROCESS

### Fermentation

The fermentation was carried out in a laboratory bioreactor for small batch studies, equipped with two 1.5 liter vessels.

Initially, a screening with various culture media was carried out, in order to identify the most suitable medium for the growth of *L. salivarius SGL03* strain (DSM 25381) in terms of biomass. The identified culture medium has the following composition:

| | |
|---|---|
| Meat peptone | 18 g/l |
| Yeast extract | 4 g/l |
| Dextrose | 60 g/l |
| Potassium dihydrogen phosphate | 2 g/l |
| Ammonium citrate tribasic | 2 g/l |
| Magnesium sulfate heptahydrate | 0.2 g/l |
| Manganese sulfate monohydrate | 0.05 g/l |
| Cysteine-HCl | 0.5 g/l |
| Tween 80 | 10 g/l |

Once filled with 1.5 liters of the selected culture medium, the vessels were sterilized in autoclave at 105°C for 90 minutes, and connected to the control panel. The set parameters for batch fermentation were as follows:
- temperature 37°C;
- pH 6.0, controlled with 5N NaOH;
- oxygen;
- stirring at 100 rpm.

The inoculum with 3% of pre-culture in exponential phase was obtained by performing a short scale up: from 10 ml of MRS to 50 ml, ending with the 1.5 liter vessel.

### Lyophilization

The fermentation process was stopped after about 7 hours from the inoculum, specifically when the optical density of the bacterial culture, measured at 600 nm, reached 10. The broth culture was then centrifuged at 9,000 rpm for 20 minutes at 10°C, the supernatant was discharged, and the pellet resuspended in a 1:1 ratio with a cryoprotector having the following composition:

| | |
|---|---|
| Trealose | 150 g/l |
| Polyvinylpyrrolidone K90 | 25 g/l |
| Potato starch | 25g/l |

The pellet mixed with the cryoprotector was then placed inside a metal container having a support surface perfectly adherent to the surface of the freeze-dryer shelf. The freeze-dryer used was a Lyobeta 3PS (Telstar, Spain). Table 5 below summarizes the lyophilization recipe used, with the relevant control parameters used during the lyophilization process.

**Table 5: L. salivarius SGL03 lyophilization parameters *temperature goes from -5°C to -55°C in 1h, and then is maintained at -55°C for 3h and 30 minutes.**

| **Lyophilization process steps** | **Temperature (°C)** | **Vacuum (µbar)** | **Time (hh:mm)** |
|---|---|---|---|
| Lyophilization tray cooling | -5 | | |
| Freezing* | -55 | | 04:30 |
| Condenser preparation | | | 00:15 |
| Vacuum in the lyophilization chamber | | 100 | |
| Primary drying | -45 | 100 | 11:30 |
| Primary drying | -20 | 100 | 10:45 |
| Primary drying | 18 | 100 | 01:30 |
| Secondary drying | 18 | 0 | 24:00 |

After the lyophilization, also *L. salivarius SGL03* (DSM 25381) percentage of vitality was evaluated, by revitalizing the freeze-dried obtained in MRS medium for about 30 minutes at 37°C, and performing a plate count.

### RESULTS

### Fermentation and Lyophilization

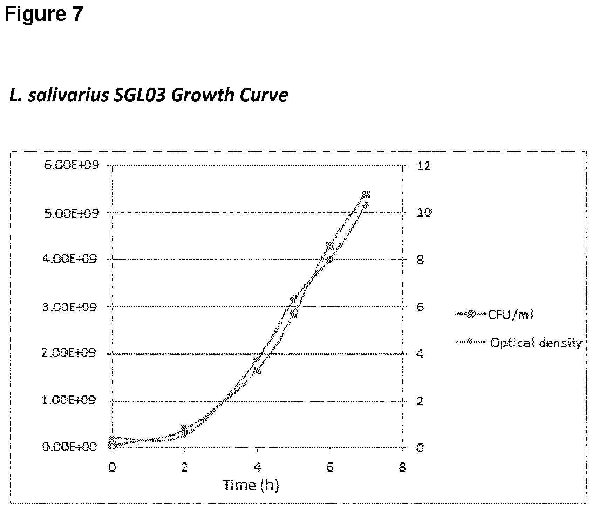

*L. salivarius SGL03* (DSM 25381) growth was monitored, in terms of optical density at 600 nm and CFU/ml, during the first 6-7 hours from fermenter inoculum, until reaching an optical density of 10. The growth curve obtained during fermentation is shown in Figure 7.

At the harvesting of the broth culture, the yield in terms of CFU was of about 2.5x10⁹CFU/liter of medium. The microbial biomass obtained after lyophilization was of about 10 g per liter of medium. After revitalization of the strain, the count result was of 200x10⁹CFU/g, therefore vitality percentage after lyophilization was 54%.

### EXAMPLE 5: PRODUCTION AD CHARACTERIZATION OF L. SALIVARIUS SGL03 SECRETOME

### Analysis of L. salivarius SGL03 Secretome

*L. salivarius* SGL03 strain (DSM 25381) was harvested from cultures grown in MRS medium by centrifugation (5,000 rpm, 10 minutes, 4°C) after 12 hours of fermentation at 37°C. The supernatant was then filtered through 0.22 µm filters (Millipore). This sample, referred to the raw cell-free supernatant, was concentrated by ultrafiltration using 3 kDa nominal molecular weight limit (cut-off) filtering units (Ultracel YM-3, Millipore), and the fraction with a cut-off lower than 10 kDa was collected and concentrated using Vivaspin 6 tubes (Sartorius Stedim Biotech GmbH, Goettingen, Germania).

The protein concentration was determined using the Bradford method (Sigma-Aldrich, St. Louis, Missouri) according to the manufacturer's instructions.

The secreted protein sample (cut-off <10 kDa) was then loaded onto SDS-PAGE gel, together with the molecular mass standards (Bio-Rad Laboratories, Hercules, California). The gel was prepared with a 5% acrylamide/bis-acrylamide packing gel, and a 5% separation gel for tricine-SDS-PAGE. The separation was performed at 25 mA for 3 hours on a vertical slab gel apparatus, Mini PROTEAN 3 (Bio-Rad). The gel was stained with Coomassie Brilliant Blue G-250.

### Antimicrobial Activity of L. salivarius SGL03 (DSM 25381) Secretome

The antimicrobial activity of the total secretome was analyzed by the diffusion in agar method. To detect the antimicrobial activity of *L. salivarius SGL03* strain (DSM 25381) secretome, the culture supernatant was centrifuged at 5,000 rpm for 10 minutes at 20°C, brought to pH 7 with 2N NaOH, and treated with 1000 U/ml of catalase (Sigma-Aldrich, St.Louis, Missouri) to exclude effects due to organic acids and hydrogen peroxide. The supernatant was then sterilized through a 0.22 µm filter (Millipore), and dilutions of the supernatant in sterile M17 medium were performed. Subsequently, 25 µl aliquots of the diluted supernatant samples were poured into 4 mm holes made on the surface of M17 agar plates (1.2% agar), previously spatulated with 1x10⁸CFU/ml of the marker strain in exponential growth phase. A 0.9% NaCl solution was also tested as a control test. The plates were incubated at 37°C for 24 hours.

The antimicrobial activity was highlighted by the presence of a growth inhibition halo, whose diameter in millimeter (mm) for the secretome was measured. For heterologous proteins, instead, the antimicrobial activity was expressed in the form of arbitrary units (AU/ml), defined as the reciprocal of the highest dilution able to produce an inhibition halo.

### RESULTS

### Analysis of L. salivarius SGL03 (DSM 25381) Secretome

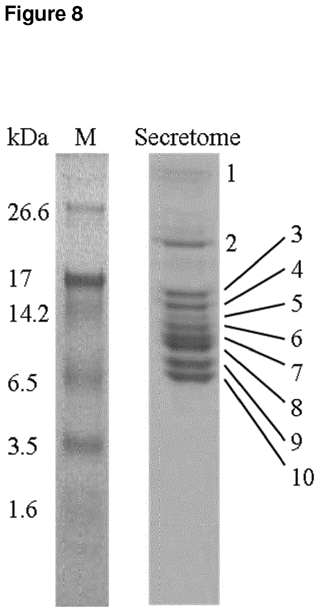

In the present study, the extracellular protein profile of *L. salivarius* SGL03 strain (DSM 25381) was analyzed by mono-dimensional electrophoresis on tricine gel. The protein concentration in the supernatant, estimated using the Bradford method, in the *L. salivarius SGL03* (DSM 25381) secretome was 8 mg/l. In order to focus on bacteriocin-like compounds, the total secretome was concentrated 15 times, and the protein fraction with a cut-off lower than 10 kDa was loaded on to SDS-PAGE gel (Figure 8). The separated bands were then extracted from the gel, and treated with trypsin before the analysis by mass spectrometry.

### Antimicrobial Activity of L. salivarius SGL03 Secretome

The secretome obtained from *L. salivarius* SGL03 strain (DSM 25381) was tested for its antibacterial activity against various Gram-positive and Gram-negative bacteria (Table 6). The antibacterial activities were determined by measuring the diameter of the inhibition halo. *L. salivarius SGL03 strain (DSM 25381)* showed inhibitory activity against *Streptococcus pyogenes, Enterococcus faecium, Streptococcus uberis.* The degree of inhibition against S. *pyogenes* was high, with a 15 mm inhibition halo diameter; and moderate against E. *faecium* and S. *uberis,* with a 6 mm inhibition halo diameter.

**Table 6: Antibacterial activity of L. salivarius SGL03 secretome**

| Indicator strains | Antibacterial activity | Inhibition zone (mm) |
|---|---|---|
| *L. innocua* ATCC 33090 | - | |
| *E. faecium* Sintal Group | + | 6 |
| S. *uberis* ATCC 700407 | + | 6 |
| *S. pyogenes* ATCC 19615 | + | 15 |
| S. *mutans* ATCC 35668 | - | |
| *L. lactis* Sintal Group | - | |
| *E. aerogeries* ATCC 13048 | - | |
| *E. cloacae* ATCC 13047 | - | |
| *E. coli* intal Group | - | |
| *E. faecalis* ATCC 49149 | - | |
| *K. xytoca ATCC49131* | - | |
| *S. typhimurium ATCC14028* | - | |
| *S. aureus ATCC 25923* | - | |
| *K. pneumoniae A TCC33495* | - | |
| *L. monocytogenes ATCC 7644* | - | |

| | | |
|---|---|---|
| Inibition zone: + inhibition activity, - no inhibition activity. | | |

### EXAMPLE 6: IDENTIFICATION OF SECRETED PROTEINS WITH ANTIMICROBIAL ACTIVITY BY MASS SPECTROMETRY (MS)

The bands were carefully cut from Coomassie gel and submitted to in-gel digestion with trypsin according to Shevchenko et al., with minor modifications. Briefly, the gel fragments were allowed to hydrate in a digestion buffer containing 50 mM NH₄HCO₃ and 12,5 ng/µl of pig trypsin (Promega, Madison, WI, USA), and the digestion was allowed to proceed at 37°C overnight. Prior to mass spectroscopy, the peptide mixtures were redissolved in 5 µl of 2% acetonitrile and 0.1% formic acid.

The peptides obtained from 5 µl of each sample were then separated by reverse phase nano-HPLC-Chip system (Agilent Technologies, Palo Alto, CA, USA) online coupled to a 3D ion trap mass spectrometer (Esquire 6000 model, Bruker Daltonics, Bremen, Germany). Sequential peptide elution was performed within the chip using a flow rate of 300 nl/minute and a linear gradient from a Solution A (2% acetonitrile; 0.1% formic acid), 50% v/v, to a Solution B (98% v/v acetonitrile; 0.1 % v/v formic acid) in 20 minutes on a Zorbax 300SB-C18 enrichment column (40 nl, 5µm) and on a Zorbax 300SB-C18 (43 mm x 75µm, 5µm) analytical column. The complete system was fully controlled by ChemStation (Agilent Technologies) and EsquireControl (Bruker Daltonics) softwares.

The scan range used to acquire the spectra was 300-1800 m/z (mass/charge ratio). For MS tandem experiments, the system was operated with an automatic switch between MS and MS/MS modes.

The three most abundant peptides for each m/z value were selected to be further isolated and fragmented. The MS/MS scan was performed in normal resolution mode, at a scan speed of 13,000 m/z per second. An average of five scans were performed to obtain a MS/MS spectrum. For the peptides identification, some searches were carried out using the search software Mascot in the National Center for Biotechnology Information (NCBInr) database containing non-redundant protein sequences (NCBInr 20.130.918 database, 32611672 sequences, 11345269536 residues).

The following parameters were used for the search: specific digestion with trypsin, up to one missed cleavage; fixed and variable modifications: cysteine carbamidomethylation and methionine oxidation; mass tolerance for peptide and fragment ± 0.9 Da and ± 0.9 Da, respectively; peptide charges: +1, +2 and +3. Proteins were identified with a significant result (P <0.05) based on the individual score for the peptide ion. These scores were automatically calculated by the Mascot program, wherein P is the probability that the match observed is a random event.

### RESULTS

A total of 10 different proteins were found in the tested media. The identified secreted proteins are summarized in Table 7 below.

**Table 7: Proteins secreted by L.salivarius SGL03 and identified by MS.**

| Protein name | band # | NCBI acc. # | # of Peptides identified | Mascot score | Sequence Coverage (%) | Mr. (Da) Exp./Theor. |
|---|---|---|---|---|---|---|
| *[Lactobacillus salivarius*] | | | | | | |
| Peptidoglycan Binding Protein | 1 | gi\|90962011 | 5 | 228 | 20 | 28000/23313 |
| 50S Ribosomal Protein L1 | 2 | gi\|385840757 | 2 | 124 | 16 | 19000/17507 |
| 30S Ribosomal Protein S5 | 3 | gi\|90962389 | 2 | 147 | 15 | 16000/17289 |
| 30S Ribosomal Protein S8 | 4 | gi\|90962392 | 8 | 270 | 47 | 15000/14676 |
| 50S Ribosomal Protein L11 | 5 | gi\|90962214 | 5 | 166 | 29 | 14000/14879 |
| 50S Ribosomal Protein L14 | 6 | gi\|90962396 | 5 | 267 | 38 | 13000/13076 |
| 50S Ribosomal Protein L27 | 7 | gi\|90961931 | 5 | 167 | 45 | 11000/9941 |
| DNA-Binding Protein HU | 8 | gi\|90962747 | 2 | 95 | 28 | 9000/9805 |
| 30S Ribosomal Protein S20 | 9 | gi\|489801430 | 3 | 68 | 18 | 8000/9126 |
| 50S Ribosomal Protein L30 | 10 | gi\|90962388 | 3 | 123 | 58 | 6500/6557 |

### EXAMPLE 7: HETEROLOGOUS EXPRESSION OF L. SALIVARIUS SGL03 SECRETOME PROTEINS AND EVALUATION OF THE ANTIMICROBIAL ACTIVITY

### Design of Primers and Amplification Conditions by PCR

Due to the fact that many ribosomal proteins are characterized by extra ribosomal features, in particular for antimicrobial functions, we have decided to concentrate initially on rpmA (50S Ribosomal Protein L27), rpmD (50S Ribosomal Protein L30), rpsT (30S Ribosomal Protein S20), and LSL_0885 (DNA-Binding Protein HU) proteins.

The primers were designed to amplify the entire encoding sequence of rpmA (Gene Bank, accession number: Q1WTI2), rpmD (Gene Bank, accession number: Y_536304), rpsT (Gene Bank, accession number: Q1WU88), and LSL_0885 (Gene Bank, accession number: Q1WTQ5) genes.

The sequence of mature polypeptides (rpmA 282 pb, rpmD 183 pb, rpsT 255 pb, LS_0885 276 pb) was amplified by PCR using the following primers:
For rpmA CTCAGCACATATGTTAATGAATTTACAATTCTTCGCT SEQ ID NO. 5
Rev rpmA CCCTCGACATATGGATAAATTAAAAGTTACTTTAATTCG SEQ ID NO. 6
For rpmD GCTGGATCCTTATTATTCAGCAACAGGGTAAACA SEQ ID NO. 7
Rev rpmD GATGGATCCCTACTATTTAGCCAATTCAACGTCC SEQ ID NO. 8
For rpsT CCACCTCATATGCCAATTATCAAATCTGCTATTAAA SEQ ID NO. 9
Rev rpsT S20 TCTGGATCCTTATTATTTAGCTAAACGAGCAGC SEQ ID NO. 10
For LSL_0885 TTGGACGCATATGGCAAACAAAGCAGCATTGATTG SEQ ID NO. 11
Rev LSL_0885 GGCGGATCCTTATTATTTAACAGCGTCTTTCAAAGA SEQ ID NO. 12

Recognition sites for Ndel and BamHI restriction endonuclease, needed for subcloning, were embedded at the 5' and 3' ends of the mature gene, respectively. Gene amplification was performed using a total volume containing 20 ng of DNA template, 0.5 µM of each primer, 2 mM Mg²⁺, 200 mM of each deoxynucleotide triphosphate, PCR buffer 1X and 2.5 units of Taq polymerase.

The following program was used for the amplification:
Hot start at 94°C for 5 minutes, 34 denaturation cycles for 40 seconds, annealing of primers at 60°C for 30 seconds, extension at 72°C for 30 seconds, followed by a final extension at 72°C for 5 minutes.

The PCR products were analyzed in 0.8% agarose gel in TBE buffer 1X, and purified from the gel using GenElute™ Gel Extraction kit (Sigma-Aldrich, St. Louis, Missouri), following the manufacturer's instructions.

### Isolation of DNA Clones from PCR Products and Sequencing

PCR products were ligated to the pGEM Teasy-vector, to generate recombinant plasmids using T4 DNA ligase. Competent cells of E. *coli* JM109 were transformed using the pGEM Teasy plasmid vector. Transformed bacteria were selected by blue-white colony screening on medium containing Ampicillin (100 µg/ml), X-Gal (80 µg/ml) and IPTG (0.5 mM).

The plasmid DNA templates were purified using Wizard® Plus SV Minipreps DNA Purification System (Promega) purification system.

RpmA, rpmD, rpsT and LSL_0885 gene sequencing was performed using the Sanger method (BMR-genomics, Padova, Italia).

### Heterologous Expression of Recombinant Proteins

*E. coli* BL21 (DE3) cells were used as transformation hosts for the expression of recombinant proteins. Such cells, containing RNA polymerase T7 gene under the control of the lacl promoter gene, were transformed using the pET-15b plasmid.

A single colony of transformed E. *coli* BL2 (DE3) cells was incubated at 37°C, under 200 rpm stirring, in 2 ml of Luria-Bertani (LB) medium, containing Ampicillin (100 µg/ml).

500 µl of culture broth were taken and inoculated in 1 liter of LB medium.

The cells were grown at 37°C, under vigorous stirring at 200 rpm, until reaching an optical density of 0.8 at 600 nm.

Isopropyl-β-D-1-thiogalactopyranoside (IPTG) was added to a final concentration of 0.5 mM to induce the expression of mature peptides in E. coli.

The incubation was then extended for 4 hours at 37°C, while keeping stirring at 200 rpm.

Aliquots were taken from the bacterial suspension at 4, 6, 12 hours.

The expressed proteins were purified by Ni-sepharose columns, following manufacturer's instructions (GE Healthcare), and then concentrated with PBS buffer at 4°C.

The degree of purity of each purified recombinant protein was evaluated by 15% SDS-PAGE, and confirmed by nano-HPCL coupled to mass spectrometry.

### Antimicrobial Activity Evaluation

In order to test the bacteriostatic and bactericidal activity of rpmA, rpmD, rpsT, and LSL_0885 against the S. *pyogenes* ATCC19615 strain, the latter was grown in 100 ml of M17 medium until the exponential phase (10⁸ CFU/ml) was reached. Four 100 ml flasks with S. *pyogenes* were produced, one for each protein to be tested. Subsequently, the proteins were filtered through 0.45 µm filters (Millipore) and singly added to the grown cultures of S. *pyogenes,* at the concentration of 133 AU/ml.

A culture of S. *pyogenes,* grown in M17 medium without any addition of the purified proteins, was used as control. After protein addition, the cultures were incubated at 37°C for another 6 hours. Changes in culture turbidity were monitored over time by recording the optical density at 600 nm, and performing CFU/ml counts on M17 agar plates.

### RESULTS

### Antimicrobial Activity Evaluation

Heterologous expression was performed to obtain purified proteins. A positive E. coli BL21 (DE3) clone allowed to accumulate the recombinant protein (i.e. rpmA, rpmD rpsT, and LSL_0885) in a cytoplasm soluble form, and produced at a constant level with IPTG.

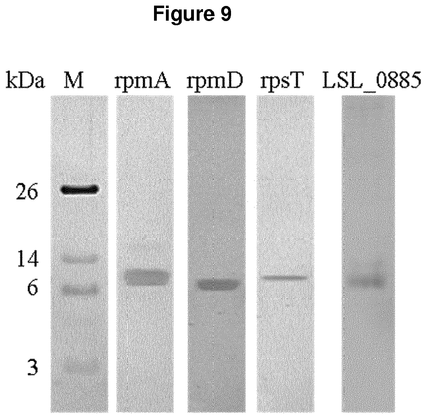

The purified proteins from heterologous expression were detected as a band of about 10, 7, 9 and 10 kDa by SDS-PAGE (Figure 9), which were identified as rpmA, rpmD, rpsT and LSL_0885, and having a Mr of 9941, 6557, 9126, and 9802Da by MS/MS analysis, respectively.

The total quantity of purified proteins was 1.5 mg/l, 0.3 mg/l, 6 mg/l, and 11 mg/l for rpmA, rpmD, rpsT, and LSL_0885, respectively.

The purified rpmA, rpmD, rpsT, and LSL_0885 proteins were tested for their antimicrobial activity against the strains reported in Table 6.

The results showed that rpmA and rpmD proteins exhibit a specific antimicrobial activity against S. *pyogenes, E. faecium* and S. *uberis* strains.

Various concentrations of the purified proteins were tested: 0.25 µg/µl, 0.5 µg/µl, 1.0 µg/µl, 1.5 µg/µl, and 2.0 µg/µl (Table 8).

**Table 8: Results of the antimicrobial activity of proteins produced by L. salivarius SGL03 against S. pyrogenes ATCC19615. Inhibition halo: + inhibitory activity, - no inhibitory activity.**

| **Protein (µg/µl)** | **50S Ribosomal Protein L27 (rpmA)** | **50S Ribosomal Protein L30 (rpmD)** | **30S Ribosomal Protein S20 (rpsT)** | **DNA HU Binding Protein(LSL_ 0885)** | **Inhibition Halo (mm)** |
|---|---|---|---|---|---|
| | **Antimicrobial Activity** | | | | |
| 0.25 | - | - | - | - | 0 |
| 0.50 | - | - | - | - | 0 |
| 1.0 | + | + | - | - | 2 |
| 1.5 | + | + | - | - | 2 |
| 2.0 | + | + | - | - | 6 |

The arbitrary unit calculated for rpmA and rpmD proteins was 133 AU/ml, using *Streptococcus pyogenes* as marker strain.

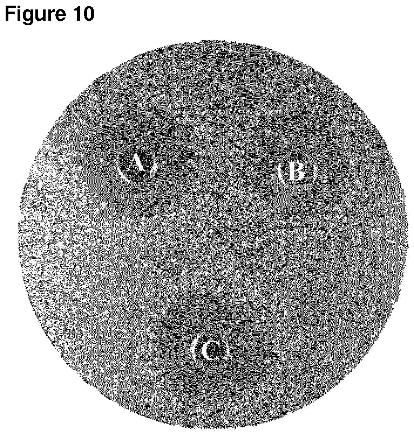

RpmA and rpmD were tested together to verify the presence of any synergistic activity. To this end, 15 µL of each protein, at a concentration of 1 µg/µl, were tested in combination, and a 0.5 mm increase of the inhibition halo diameter was obtained using the agar diffusion method (Figure 10).

The addition of purified rpmA and rpmD proteins, at a concentration of 133 AU/mL, to the S. *pyogenes* ATCC 19615 culture, in the logarithmic growth phase, resulted in a rapid decrease of S. *pyogenes* viable cells from 10⁸ CFU/ml to 10⁶ CFU/ml and from 10⁸ CFU/ml to 10⁵ CFU/ml, over a period of 9 hours, in cultures treated with rpmA and rpmD, respectively.

In the control sample (no addition of purified proteins), S. *pyogenes* ATCC 19615 viable cells reached 10¹⁰ CFU/ml after 9 hours growth.

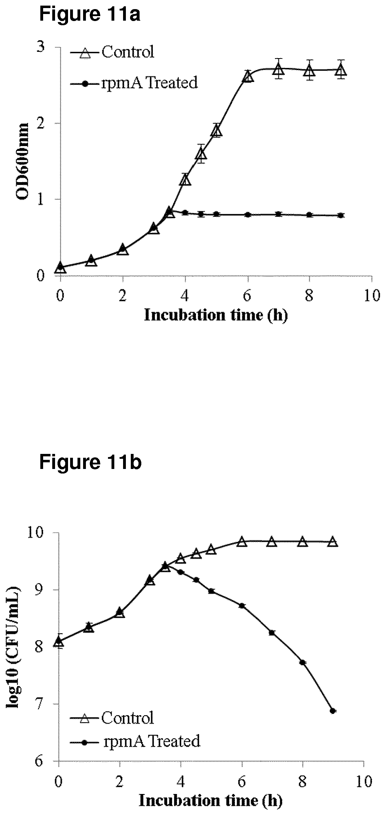

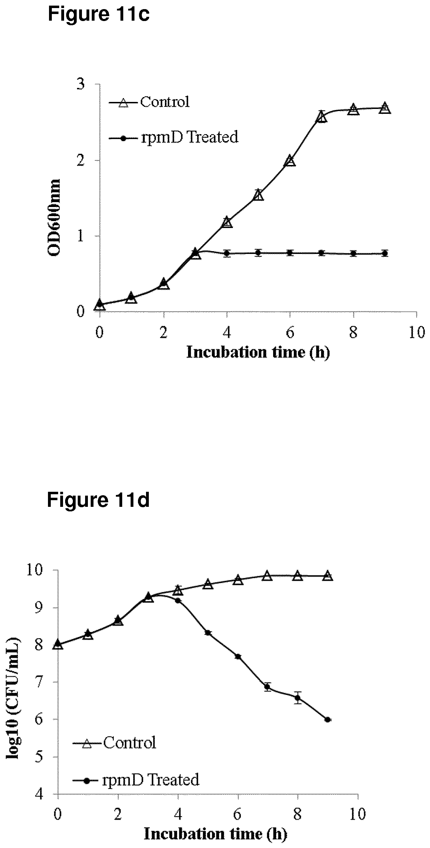

The optical density of the marker microorganism remained constant after the addition of purified rpmA and rpmD proteins (Figure 11a, 11b, 11c and 11d). These results showed that both proteins exhibit bactericidal activity against S. *pyogenes.* From the detailed description and Examples above, the advantages obtained with the probiotic strain of the present invention are apparent. Specifically, such probiotic strain showed to be surprisingly and advantageously suitable for the production of proteins having inhibitory activity against certain Gram-positive bacteria.

## Claims

1. Use of the *L. salivarius SGL03* DSM 25381 strain as an antimicrobial agent.

2. The use according to claim 1, wherein said strain has an inhibitory action against Gram positive bacteria.

3. The use according to any one of claims from 1 to 2, wherein said strain has an inhibitory action against *Streptococcus pyogenes, Enterococcus faecium, Streptococcus uberis.*

4. The use according to any one of claims from 1 to 3, wherein said strain comprises proteins chosen from the group consisting of Peptidoglycan Binding Protein, 50S Ribosomal Protein L1, 30S Ribosomal Protein S5, 30S Ribosomal Protein S8, 50S Ribosomal Protein L11, 50S Ribosomal Protein L14, 50S Ribosomal Protein L27, DNA-Binding Protein HU, 30S Ribosomal Protein S20, and 50S Ribosomal Protein L30.

5. The use according to claim 4, wherein said proteins are chosen from the group consisting of 50S Ribosomal Protein L27, DNA-Binding Protein HU, 30S Ribosomal Protein S20, and 50S Ribosomal Protein L30.

6. A probiotic composition comprising *L. salivarius SGL03* DSM 25381 strain supplemented with one or more antimicrobial proteins chosen from the group consisting of 50S Ribosomal Protein L27, DNA-Binding Protein HU, 30S Ribosomal Protein S20, and 50S Ribosomal Protein L30.

7. The composition according to claim 6, wherein said one or more antimicrobial proteins are in an amount in a range from 50 to 100µg of purified protein.

8. The composition according to anyone of claims 6 or 7, comprising magnesium peroxide.

9. Use of the composition according to any one of claims 6, 7 or 8, for the preparation of a nutraceutical product.

10. The use according to claim 9, wherein said nutraceutical product is in the form of a capsule, a tablet, a powder, or a chewing gum.

11. Use of the composition according to anyone of claims 6, 7 or 8 for the preparation of a dermo-cosmetic product.

12. The use according to claim 11, wherein said demo-cosmetic product is in the form of a patch, a plaster, a cream, a powder or talk, oil, or dry oil.

## Patentansprüche

1. Verwendung des Stammes *L. salivarius SGL03* DSM 25381 als antimikrobielles Mittel.

2. Verwendung nach Anspruch 1, wobei der Stamm eine hemmende Wirkung gegen grampositive Bakterien aufweist.

3. Verwendung nach irgendeinem der Ansprüche von 1 bis 2, wobei der Stamm eine hemmende Wirkung gegen *Streptococcus pyogenes, Enterococcus faecium, Streptococcus uberis aufweist.*

4. Verwendung nach irgendeinem der Ansprüche von 1 bis 3, wobei der Stamm Proteine umfasst, die ausgewählt sind aus der Gruppe bestehend aus Peptidoglycan-Bindungsprotein, 50S-ribosomalem Protein L1, 30S-ribosomalem Protein S5, 30S-ribosomalem Protein S8, 50S-ribosomalem Protein L11, 50S-ribosomalem Protein L14, 50S-ribosomalem Protein L27, DNA-bindendem Protein HU, 30S-ribosomalem Protein S20 und 50S-ribosomalem Protein L30.

5. Verwendung nach Anspruch 4, wobei die Proteine ausgewählt sind aus der Gruppe bestehend aus 50S-ribosomalem Protein L27, DNA-bindendem Protein HU, 30S-ribosomalem Protein S20 und 50S-ribosomalem Protein L30.

6. Probiotische Zusammensetzung, umfassend einen Stamm von L. *salivarius SGL03* DSM 25381, supplementiert mit einem oder mehreren antimikrobiellen Proteinen, ausgewählt aus der Gruppe bestehend aus 50S-ribosomalem Protein L27, DNA-bindendem Protein HU, 30S-ribosomalem Protein S20 und 50S-ribosomalem Protein L30.

7. Zusammensetzung nach Anspruch 6, wobei das eine oder die mehreren antimikrobiellen Proteine in einer Menge in einem Bereich von 50 bis 100µg gereinigtem Protein vorliegen.

8. Zusammensetzung nach irgendeinem der Ansprüche 6 oder 7, umfassend Magnesiumperoxid.

9. Verwendung der Zusammensetzung nach irgendeinem der Ansprüche 6, 7 oder 8 zur Herstellung eines nutrazeutischen Produkts.

10. Verwendung nach Anspruch 9, wobei das nutrazeutische Produkt in Form einer Kapsel, einer Tablette, eines Pulvers oder eines Kaugummis vorliegt.

11. Verwendung der Zusammensetzung nach irgendeinem der Ansprüche 6, 7 oder 8 zur Herstellung eines dermokosmetischen Produkts.

12. Verwendung nach Anspruch 11, wobei das dermokosmetische Produkt in Form eines Patches, eines Pflasters, einer Creme, eines Pulvers oder eines Talkpuders, eines Öls oder eines Trockenöls vorliegt.

## Revendications

1. Utilisation d'une souche de *L. salivarius SGL03* DSM 25381 en tant qu'agent antimicrobien.

2. Utilisation selon la revendication 1, dans laquelle ladite souche a une action inhibitrice contre des bactéries Gram positives.

3. Utilisation selon l'une quelconque des revendications de 1 à 2, dans laquelle ladite souche a une action inhibitrice contre *Streptococcus pyogenes, Enterococcus faecium, Streptococcus uberis.*

4. Utilisation selon l'une quelconque des revendications de 1 à 3, dans laquelle ladite souche comprend des protéines choisies parmi le groupe constitué de Protéine liant les peptidoglycanes, 50S Protéine ribosomique L1, 30S Protéine ribosomique S5, 30S Protéine ribosomique S8, 50S Protéine ribosomique L11, 50S Protéine ribosomique L14, 50S Protéine ribosomique L27, Protéine liant l'ADN HU, 30S Protéine ribosomique S20, et 50S Protéine ribosomique L30.

5. Utilisation selon la revendication 4, dans laquelle lesdites protéines sont choisies parmi le groupe constitué de 50S Protéine ribosomique L27, Protéine liant l'ADN HU, 30S Protéine ribosomique S20, et 50S Protéine ribosomique L30.

6. Composition probiotique comprenant une souche de *L. salivarius SGL03* DSM 25381 additionnée d'une ou de plusieurs protéines antimicrobiennes choisies parmi le groupe constitué de 50S Protéine ribosomique L27, Protéine liant l'ADN HU, 30S Protéine ribosomique S20, et 50S Protéine ribosomique L30.

7. Composition selon la revendication 6, dans laquelle lesdites une ou plusieurs protéines antimicrobiennes sont dans une quantité comprise dans une plage de 50 à 100 µg de protéine purifiée.

8. Composition selon l'une quelconque des revendications 6 ou 7, comprenant du peroxyde de magnésium.

9. Utilisation de la composition selon l'une quelconque des revendications 6, 7 ou 8, pour la préparation d'un produit nutraceutique.

10. Utilisation selon la revendication 9, dans laquelle ledit produit nutraceutique est sous forme d'une capsule, d'un comprimé, d'une poudre, ou d'un chewing-gum.

11. Utilisation de la composition selon l'une quelconque des revendications 6, 7 ou 8 pour la préparation d'un produit dermo-cosmétique.

12. Utilisation selon la revendication 11, dans laquelle ledit produit dermo-cosmétique est sous forme d'un patch, d'un pansement, d'une crème, d'une poudre ou de talc, d'huile, ou d'huile sèche.
